# EUROPÄISCHE PATENTSCHRIFT

(11) **EP 1 102 839 B1**
(45) Veröffentlichungstag und Bekanntmachung des Hinweises auf die Patenterteilung: **02.10.2002**
(21) Anmeldenummer: 99931239.0
(22) Anmeldetag: 02.07.1999
(51) Int. Cl.: C12N 11/02, C12P 41/00, C08G 18/32

(54) **ENZYMHALTIGE POLYMERE**
ENZYME-CONTAINING POLYMERS
POLYMERES CONTENANT UNE ENZYME

(30) Priorität: 21.07.1998 DE 19832547
(43) Veröffentlichungstag der Anmeldung: 30.05.2001
(73) Patentinhaber: BASF AKTIENGESELLSCHAFT, 67056 Ludwigshafen (DE)
(72) Erfinder: STÜRMER, Rainer, D-67127 Rödersheim-Gronau (DE)
(86) Internationale Anmeldenummer: EP9904590
(87) Internationale Veröffentlichungsnummer: WO00005354

(56) Entgegenhaltungen:
- DE-A- 19 505 672
- US-A- 3 672 955
- US-A- 4 634 671
- US-A- 5 482 996

## Beschreibung

Die vorliegende Erfindung betrifft neue enzymhaltige Polymere, sowie die Herstellung und Verwendung der enzymhaltigen Polymere.

Die enzymhaltigen Polymere werden als Enzymkatalysatoren in chemischen Reaktionen eingesetzt. Gegenüber den freien Enzymen zeichnen sich die immobilisierten Enzyme durch eine erhöhte Stabilität und Standzeit unter kontinuierlicher und diskontinuierlicher Reaktionsführung sowie durch eine leichte Rückgewinnung der katalytisch aktiven Spezies bei diskontinuierlichen Reaktionsansätzen aus.

Es ist bekannt, Enzyme durch kovalente Bindung unter Erhalt der Aktivität in Polymere einzubauen. Weiterhin ist bekannt, Polyurethane, Polyharnstoffe und Polyamide als polymeres Trägermaterial zu verwenden. US 5,482,996 beschreibt proteinhaltige Polyurethane, Polyharnstoffe, Polyamide und Polyester. Dabei werden die Proteine durch Anbindung eines amphiphilen Spacers (Polyalkylenoxid), der die für die Polymerisationsart geeignete endständige funktionelle Gruppe trägt, vom wässrigen Lösungsmittelsystem in das organische Lösungsmittelsystem unter Erhalt der Aktivität übertragen. Die Umsetzung mit den Monomeren erfolgt anschließend mit der funktionellen Gruppe des Spacers. Diese Methode ermöglicht nur einen geringen Belegungsgrad und ist zudem sehr aufwendig und damit kostenintensiv, da die Enzyme nicht direkt in das Polymer eingebaut werden, sondern die Anknüpfung der amphiphilen Spacereinheit als zusätzlicher Schritt vorgeschaltet wird.

US 3,672,955 lehrt die Herstellung von in Polyurethan immobilisierten Enzymen, wobei zunächst mit Polyether- und Polyesterpolyol-Einheiten verbrückte, amphiphile Isocyanat-Präpolymere hergestellt werden und diese in einem nicht mit Wasser mischbaren Lösungsmittel mit der wäßrigen Enzymlösung emulgiert und zur Reaktion gebracht werden. Durch den Kontakt mit Wasser werden die nicht mit den funktionellen Gruppen der Enzyme abreagierten Isocyanate in instabile Carbaminsäure-Gruppen überführt, die anschließend unter Eliminierung von CO₂ in die entsprechende Amine zerfallen. Die entstehenden Amino-Gruppen reagieren mit noch vorhandenen Isocyanat-Gruppen unter Vernetzung, das entstandene CO₂ führt zur Aufschäumung der Polymermasse. US 4,342,834 verfolgt die analoge Präpolymer-Strategie der Überführung der Polymerisation in wässerige Systeme, wobei im Unterschied zu US 3,672,955 die Reaktion vollständig in wäßriger Lösung durchgeführt wird.

Die Verwendung der, analog mit Polyalkylenoxid-Einheiten verbrückten, amphiphilen Präpolymere Hypol® (entsteht durch Reaktion eines Polyether- oder Polyesterpolyol mit Polyisocyanaten in der Gegenwart von verknüpfenden Reagenzien) und PU-3® (mit zwei endständigen TDI-Einheiten abgesättigtes Präpolymer, enthaltend Polyethylenoxid und Polypropylenoxid-Einheiten als Spacer) zur Immobilisierung einer Reihe von Enzymen aus wäßriger Lösung, wurde in wissenschaftlichen Aufsätzen beschrieben.

Mit diesen amphiphilen Präpolymeren konnten aus wäßriger Lösung Phosphotriesterasen zum Abbau von Nervengiften immobilisiert werden (K.E. Lejeune et al., Biotechnology and Bioengineering 1997, 54, 105-114). Aus wäßriger Lösung mit Präpolymeren immobilisierte Lipasen wurden zur Acylierung (S.F. Dias et al., Biocatalysis 1991, 5, 21-34.) und zur enantioselektiven Acylierung (S. Koshiro et al., Journal of Biotechnology 1985, 2, 47-57) von Alkoholen in organischer Lösung verwendet.

Bei all diesen Verfahren wird die Polymerisation (Immobilisierung des Enzyms) in Anwesenheit von Wasser mit Präpolymeren durchgeführt. Dies hat mehrere Nachteile:
- Die amphiphilen Präpolymere müssen getrennt hergestellt werden.
- Mit den enzymhaltigen Polymeren des Standes der Technik werden nur geringe Standzeiten erreicht.
- Es werden nur geringe Beladungsdichten mit aktiver Enzymspezies erreicht. Die in den obengenannten Arbeiten typische Beladung eines Polymeren mit Enzym liegt bei max. 1 % in Relation zur Gesamtmasse. Daraus resultieren geringe Raum-Zeit-Ausbeuten (RZA).

Der Erfindung lag daher die Aufgabe zugrunde, den geschilderten Mängeln abzuhelfen und neue, durch ein vereinfachtes Verfahren hergestellte, enzymhaltige Polymere mit optimierten Eigenschaften. wie höherer Standzeit und einer höheren Beladungsdichte an katalytisch aktiver Enzymspezies, bereitzustellen.

Es wurde gefunden, daß man die erfindungsgemäßen enzymhaltigen Polymere besonders vorteilhaft in einem vereinfachten, wasserfreien Verfahren durch direkte Umsetzung des Enzyms in organischer Lösung mit quervernetzenden, endständig reaktive funktionelle Gruppen tragenden, organischen Verbindungen erhält. Dabei setzt man das Enzym in einem organischen Lösungsmittel im ersten Schritt mit dem mindestens bifunktionellen Monomer der Formel I

R¹(X)ₛ(Y)ₜ I

um, wobei
- R¹: eine 2- bis 5-fach gebundene Alkan-, Alken-, Cycloalkan-, Cycloalken-, Aryl-, Arylalkan-, Diarylenether-, Diarylenthioether- oder Diarylamingruppe darstellt, wobei die cyclischen aromatischen oder nichtaromatischen Gruppen ihrerseits durch ein bis vier C₁-C₄-Alkyl-, C₁-C₄-Halogenalkyl- oder Halogenreste substituiert sein können,
und
- X: die funktionelle Gruppe Isocyanat (-NCO) und
- Y: die funktionelle Gruppe Isothiocyanat (-NCS)
mit s ≥ 0 und t ≥ 0 und 5 ≥ s+t ≥ 2
oder
- X=Y: die funktionelle Gruppe -COR³
mit 5 ≥ s+t ≥ 2 darstellt,
worin R³ eine durch die Amino-, Hydroxy- oder Thio- Funktionalität des Enzyms verdrängbare Abgangsgruppe darstellt
und gibt in einem 2. Schritt ein mindestens bifunktionelles Amin oder ein Gemisch mindestens bifunktioneller Amine zu.

Die Enzyme sind als mindestens monofunktionelle, in der Regel polyfunktionelle Amine, Alkohole und/oder Thiole zu sehen. Sie können beispielsweise aus Organismen wie Pilzen, Bakterien (gram⁺ oder gram⁻), Hefen oder Mammalia gewonnen werden und weisen in den organischen Lösungsmitteln eine enzymatische Aktivität auf.

Beispielhaft seien Hydrolasen wie Esterasen, Lipasen, Amidasen, Proteasen sowie Haloperoxidasen, Aminotransferasen, Aspartat aminotransferasen, Pyruvatdecarboxylasen, Lyasen/Laccasen, Benzol dioxygenäse, Aspartasen, Dehydrogenasen, Fumarasen, Dehalogenasen, Aminosäuredehydrogenasen, Oxygenasen, Aminopeptidasen, Aminoamidasen, Alkylaminopeptidasen und Racemasen genannt.

Bevorzugt sind Lipasen aus *Aspergillus niger, Aspergillus oryzae, Candida antarctica, Candida cylindracea, Candida lipolytica, Candida utilis, Candida rugosa, Mucor javanicum, Mucor miehei, Rhizomucor miehei, Rhizopus arrhizus, Rhizopus delemar, Rhizopus niveus, Penicillin acylase, Penicillin roqueforti, Thermus aquaticus, Thermus flavus, Thermus thermophilus, Chromobacterium viscosum, Pseudomonas putida, Pseudomonas fluorescens, Pseudomonas cepacia,*
sowie Schweinepankreaslipase (PPL) und Weizenkeimlipase, Esterasen aus Bacterium subtilis, Bacterium stearothermophilus, Bacterium thermoglucosidasius, Candida lipolytica, Mucor Miehei, Pferdeleber, Schweineleber, Saccharomyces cerevisiae, Thermoanaerobium brockii, Elektrophorus electricus, Proteasen aus *Subtilisin, Thermolysin, Bacterium subtilis, Subtilisin carlsberg, Thermolysin, Nagarse, Tritirachium alba, Aspergillus oryzae, Aspergillus sp.,*
Benzoldioxygenase aus *Pseudomonaden, Alcaligenes sp., Micrococcus sp., Pseudomonas oleovorans,*
Dehalogenasen aus *Pseudomonas putida,*
Oxygenasen aus *Pseudomonas oleovorans, Corynebacterium equi, Nocardia carallina, Mycobacter, Xanthobacter,*
Aminoamidasen und Alkylaminopeptidasen aus *Mycobacterium,* Dehydrogenasen aus *Pseudomonas putida*
und Nitrilasen und Nitrilhydratasen aus
Aspergillus Niger JCM 1925, Fusarium sp. MY-2, Rhodococcus rhodocrus J 1, K22, PA 34 und NCIB 11216, Pseudomonas chlororaphis B 23, Corynebacterium sp. N-774.

Besonders bevorzugt ist die Lipase aus *Burgholderi plantarii.*

R¹ stellt eine die funktionellen Gruppen tragende, 2- bis 5-fach gebundene Alkan-, Alken-, Cycloalkan-, Cycloalken-, Aryl-, Arylalkan-, Diarylenether-, Diarylenthioether- oder Diarylamingruppe dar, wobei die cyclischen aromatischen oder nichtaromatischen Gruppen ihrerseits durch ein bis vier C₁-C₄-Alkyl-, wie Methyl, Ethyl, Propyl, Isopropyl, n-Butyl, iso-Butyl oder t-Butyl und/oder C₁-C₄-Halogenalkyl-, wie Trichlormethyl, Trifluormethyl oder Trifluorethyl und/oder Halogenreste, wie Cl, Br, I oder F substituiert sein können. Bevorzugte Reste R¹ sind die den entsprechenden, aus der Polyurethanchemie bekannten Polyisocyanat- bzw. Thioisocyanat-Monomeren zugrunde liegenden organischen Grundkörper. Im einzelnen seien besonders bevorzugt folgende Strukturen aufgezählt, wobei die Verknüpfungsstellen durch einen Bindungsstrich gekennzeichnet sind:

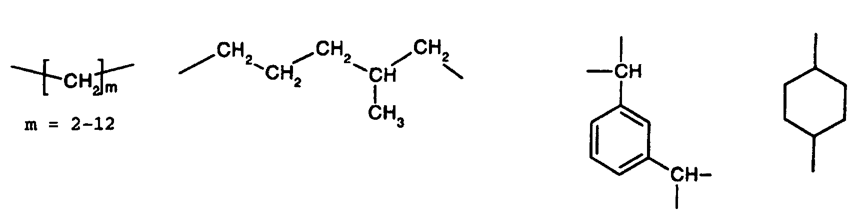

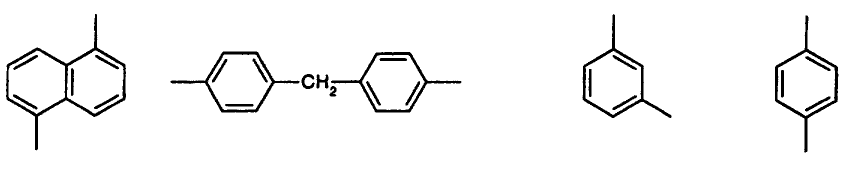

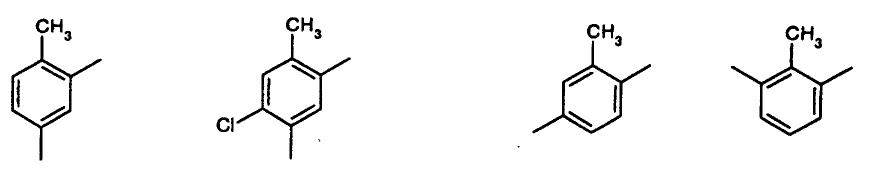

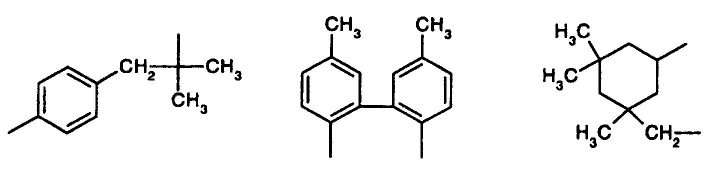

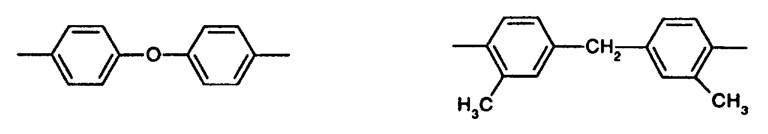

R² stellt eine, die Amino-Gruppen tragende, 2- bis 5-fach gebundene Alkan-, Alken-, Cycloalkan-, Cycloalken-, Aryl-, Arylalkan-, Dialkylenether-, Dialkylenthioether-, Diarylenether-, Diarylenthioether-, Diarylamin- oder Piperazinyldialkandiylgruppe dar, wobei die cyclischen aromatischen oder nichtaromatischen Gruppen ihrerseits durch ein bis vier C₁-C₄-Alkyl-, C₁-C₄-Halogenalkyl- und/oder Halogenreste, z.B. wie vorstehend bei R¹ genannt, substituiert sein können.

Bevorzugte Reste sind die den entsprechenden, aus der Polyurethan- und Polyamidchemie bekannten Polyamin-Monomeren zugrunde liegenden organischen Grundkörper. Im einzelnen seien besonders bevorzugt folgende Strukturen aufgezählt:

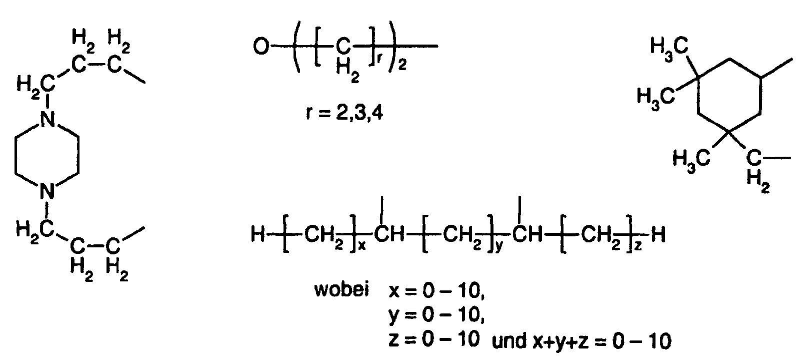

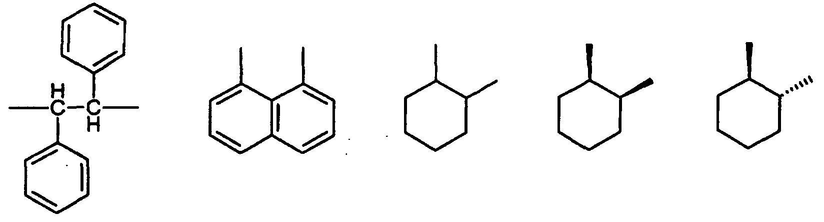

Je nach Einbau von primären oder sekundären mindestens bifunktionellen Aminen stellen R⁴ und R⁵ unabhängig voneinander Wasserstoff oder einen C₁-C₄-Alkyl-Rest, wie Methyl, Ethyl, Propyl, Isopropyl, n-Butyl, iso-Butyl, t-Butyl bzw. Aryl- oder Alkylarylrest, wie Phenyl, oder durch C₁-C₄ substituiertes Phenyl, wie Tolyl, dar.

Die Gewichtsanteile des Enzyms im polymeren Träger liegen vorzugsweise zwischen 0,01 Gew.-% und 50 Gew.-%, besonders bevorzugt zwischen 1 Gew.-% und 10 Gew.-% bezogen auf die Gesamtmasse. Wie Beispiel 3 zeigt, nimmt bei einer geringeren Beladung (0,5 %) als 1 % die Enzymaktivität und die Standzeit ab. Dahingegen führt eine höhere Beladung (5 %) als 1 % zu keiner Abnahme der Enzymaktivität und der Standzeit. Durch die höhere Beladung läßt sich eine höhere Raum-Zeit-Ausbeute (RZA) in nachstehend beschriebenen enzymkatalytischen Reaktionen erreichen.

Unter organischen Lösungsmitteln werden erfindungsgemäß aprotische Lösungsmittel, wie aliphatische, aromatische gegebenenfalls halogenierte Kohlenwasserstoffe sowie Ether verstanden. Bevorzugte organische Lösungsmittel sind solche, die gegenüber den mindestens bifunktionellen Monomeren inert sind, die mindestens bifunktionellen Monomere lösen und das Enzym nicht denaturieren. Besonders bevorzugte organische Lösungsmittel sind Toluol, Benzol, Chloroform, Methylenchlorid, Hexan, Heptan, Diethylether und Methyl-t-Butylether (MTBE), Dioxan, THF und halogenierte Aromaten wie Chlorbenzol.

Die Enzyme werden in einem organischen Lösungsmittel suspendiert und in einem ersten Schritt mit einem mindestens bifunktionellen Monomer umgesetzt. Das Monomer trägt als funktionelle Gruppen mindestens zwei, maximal fünf, besonders bevorzugt zwei oder drei Isocyanat- und/oder Thioisocyanat- bzw. mindestens 2, maximal fünf, besonders bevorzugt zwei oder drei Aktivestergruppen der Formel -COR³, worin R³ eine durch die Amino-, Hydroxy- oder Thio-Funktionalität des Enzyms verdrängbare Abgangsgruppe darstellt. Beispielhaft seien als Aktivestergruppen und damit als Acylüberträger funktionalisierte Gruppen Carbonsäurechloride, Carbonsäureanhydride, Carbonsäureester, wie Carbonsäure-N-hydroxysuccinimide, Phenolester und Halophenolester wie Pentafluorphenolester und Trichlorphenolester genannt. Als Abgangsgruppe R³ seien beispielhaft dementsprechend Halogenide wie Cl⁻, Br⁻, I-, Carboxylate wie Acetate und Propiate oder Alkoholate wie Succinimid-N-Hydroxylat, Phenolate, Halophenolate wie Pentafluorphenolat, Trichlorphenolate oder Benzotriazol-1-Hydroxylate genannt. Die funktionellen Gruppen der Enzymoberfläche (Amino-, Hydroxyl- und/oder Thiolgruppen) reagieren mit den endständigen Isocyanat- und/oder Thioisocyanat-Funktionen der Monomere unter Ausbildung von Urethan-, Thiocarbamidsäure-O-ester-(Thiourethan), Harnstoff-, Thioharnstoff-, Thiocarbamidsäure-S-esterund/oder Dithiocarbamidsäure-di-esterbindungen. Analog dazu bilden sich bei der Verwendung der polyfunktionellen Aktivester-Monomere mit den funktionellen Gruppen der Enzymoberfläche (Amino-, Hydroxyl- und/oder Thiol-Gruppen) Amid-(Jerry March, Advanced Organic Chemistry, 1992, 4. Auflage, John Wiley & Sons, New York, S. 417-425), Ester-(Jerry March, Advanced Organic Chemistry, 1992, 4. Auflage, John Wiley & Sons, New York, S. 392-398), und/oder Thiolcarbonsäureester-Bindungen (Houben-Weyl, Band IX, 4. Auflage, S.753-756). Je nach Vorhandensein und Anzahl der jeweiligen funktionellen Gruppe an der Enzymoberfläche und dem verwendeten Monomer können diese Bindungstypen beliebig kombiniert auftreten.

Als Diisocyanat-, Polyisocyanat-, Dithioisocyanat-, Polythioisocyanat-, gemischte Polyisocyanate/Thioisocyanat- oder bi- bis pentafunktionelle Aktivester-Monomere werden Alkyl-, Alken-, Alkin-, Cycloalkyl-, Cycloalken-, Aryl-, Alkylaryl-, Arylalkyl-, Diarylen-ether-, Diarylenthioether- oder Diarylamin-Verbindungen verwendet, wobei in diesen Verbindungen die cyclischen aromatischen oder nichtaromatischen Gruppen ihrerseits durch ein bis vier C₁-C₄-Alkyl-, C₁-C₄-Halogenalkyl- und/oder Halogenreste substituiert sein können. Bevorzugte Reste sind die aus der Polyurethanchemie bekannten Diisocyanate, Polyisocyanate, Dithioisocyanate, Polythioisocyanate und gemischten Polyisocyanate/Polythioisocyanate oder polyfunktionelle Carbonsäure- bzw. Carbonsäureester-Derivate die den zu den beschriebenen Polyisocyanaten analogen Grundkörper aufweisen und anstatt der Isocyanat- oder Thioisocyanat die vorstehend beschriebenen Aktivestergruppen als funktionelle Gruppen tragen. Besonders bevorzugte Monomere sind
1,4-Diisocyanatobutan,
1,6-Diisocyanatohexan,
1,8-Diisocyanatooktan,
1,12-Diisocyanatododekan,
1,5-Diisocyanato-2-methyl-pentan,
1,3-bis-Isocyanatomethyl-benzen,
trans-1,4-Diisocyanato-cyclohexan,
1,5-Diisocyanatonaphthalin,
4,4'-Methylendiphenyldiisocyanat,
1-(p-isocyanato-phenyl)-2-isocyanato-2-methyl-propan,
Toluol-2,4-diisocyanat (4-Methyl-m-phenylendisocyanat),
Toluol-2,5-diisocyanat,
Toluol-2,6-diisocyanat,
1,3-Diisocyanato-benzen,
1,4-Diisocyanato-benzen (p-Phenylendiisocyanat),
4,4'-Biphenylenoxy-diisocyanat,
3,3'-bis-Tolyl-4,4'diisocyanat,
3,3'-Dimethyl-4,4'diisocyanato-diphenylmethan,
2,4- Diisocyanato-5-chlor-toluol,
Isophorondiisocyanat
sowie die analogen Polythioisocyanate oder analogen gemischten Polyisothiocyanate/Isocyanate.

Für den Fall, daß es vorteilhaft ist, das enzymhaltige Polymer als Schaum zu erhalten (z.B. zur Erhöhung der inneren Oberfläche), können gleichzeitig mit der Zugabe des Monomers physikalische Treibmittel, wie Kohlenwasserstoffe, insbesondere Methan, Ethan, Ethylen, Propan, Propylen, Pentan, Cyclopentan, Cyclohexan oder halogenierte Kohlenwasserstoffe, insbesondere Trichlorfluormethan, Chlormethan, Dichlordifluormethan, Dichlorfluormethan, Chlordifluormethan, Chlorethan, Dichlortetrafluorethan, Octafluorcyclobutan, Hexafluorpropan, 1,1-Difluor-2,2-dichlorethan, 1,2-Difluor-1,2 dichlorethan, 1,1-Dichlorethan, Trichlorethan, Tetrachlorethan, 1-Fluor-1,2,2-trichlorethan, 1-Bromethan oder 1,1,2-Trifluor-2-chlorethan, zugesetzt werden oder Inertgase wie Stickstoff, Argon, Kohlendioxid, oder Luft während der Reaktion eingegast werden.

Die Temperatur der Reaktion ist nicht kritisch, ist aber nach oben durch die Temperaturempfindlichkeit des jeweiligen Enzyms begrenzt. Der bevorzugte Temperaturbereich liegt zwischen -30°C und 60°C, besonders bevorzugt zwischen -10°C und 10°C.

In einem zweiten Schritt werden mindestens bifunktionelle Amine oder Gemische mindestens bifunktioneller Amine zugegeben. Die Zeit zwischen Schritt 1 und 2 beträgt bevorzugt nicht länger als 1 s bis 10 min, besonders bevorzugt 30 s bis 5 min.

Die zugegebenen mindestens bifunktionellen Amine reagieren mit noch nicht abreagierten funktionellen Gruppen der Monomere unter Vernetzung und/oder Kettenverlängerung. Als mindestens bifunktionelle Amine werden primäre oder sekundäre Amine wie Alkyl-, Alken-, Alkin-, Cycloalkyl-, Cycloalken-, Aryl-, Alkylaryl-, Arylalkyl-, Diarylen-ether-, Diarylenthioether-, Diarylamin-, Dialkylamin-, Diarylthioether- oder Piperazinyldialkyl-Amine verwendet, wobei in diesen Aminen die cyclischen aromatischen oder nichtaromatischen Gruppen ihrerseits durch ein bis vier C₁-C₄-Alkyl-, C₁-C₄-Halogenalkyl- und/oder Halogenreste substituiert sein können. Bevorzugte Reste sind die aus der Polyurethan- und Polyamidchemie bekannten Amin-Monomere.

Besonders bevorzugt sind
bis-3-Aminopropyl-1,4-piperazin,
bis-3-Aminopropyl-ether,
bis-3-Aminoethyl-ether,
N-Phenylethylendiamin,
N,N'-diethyl-ethylendiamin,
N,N'-diethyl-propylendiamin,
N,N'-diethyl-butylendiamin,
N,N'-diethyl-hexylendiamin,
Isophorondiamin,
1,2-Diaminocyclohexan (eis und trans),
1,2-Diamino-1,2-diphenylethan (cis und trans),
1,8-Diamino-naphthalin,
aliphatische Amine der Formel II 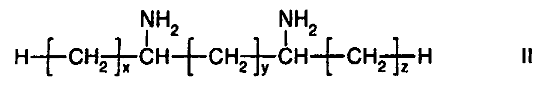 mit
x = 0 - 10
y = 0 - 10
z = 0 - 10
und 0 ≤ x+y+z ≤ 10,
wie 1,2-Diaminobutan,
1,n-Diaminoalkane, wobei unter 1,n-Diaminoalkane lineare C₂-C₁₂-Diaminoalkane mit endständigen Aminofunktionen zu verstehen sind
(n = 2 bis 12; ≅ x = 0 und z = 0 und y = 0 bis 10), wie
1,2-Diaminoethan,
1,3-Diaminopropan,
1,4-Diaminobutan,
1,5-Diaminopentan,
1,6-Diaminohexan,
1,7-Diaminoheptan,
1,8-Diaminooktan,
1,9-Diaminononan,
1,10-Diaminodekan,
1,11-Diaminoundekan oder
1,12-Diaminododekan,
sowie die jeweils N-monoalkylierten oder N-monoarylierten 1,n-Diaminoalkane (n = 2 bis 12),
und die N-alkylierten und N'-arylierten, N,N'-dialkylierten oder N,N'-diarylierten 1,n-Diaminoalkane (n= 2 bis 12).

Die Wahl der mindestens bifunktionellen Monomere und der mindestens bifunktionellen Amine hat keinen wesentlichen Einfluß auf die Ausbeute der enzymhaltigen Polymere. Daher können beliebige mindestens bifunktionelle Monomere und beliebige, mindestens bifunktionellen Amine sowie beliebige Kombinationen verwendet werden.

Besonders gute Polymer- und Katalysatoreigenschaften, wie erhöhte Standzeit und erhöhte Umsätze erhält man, wenn man, wie in Beispiel 2 ersichtlich, als mindestens bifunktionelles Monomere im ersten Schritt und als mindestens bifunktionelles Amin im zweiten Schritt bevorzugt folgende Kombinationen verwendet:
p-Phenylendiisocyanat mit 1,n-Diaminoalkan,
4-Methyl-m-phenylen-diisocyanat mit 1,n-Diaminoalkan,
4,4'-Methylen-bis-phenyldiisocyanat(MDI) mit 1,n-Diaminalkan,
p-Phenylendiisocyanat mit N-Phenylethylendiamin,
p-Phenylendiisocyanat mit 1,6-Diaminohexan,
4-Methyl-m-phenylen-diisocyanat mit N,N'-Diethyl-ethylendiamin oder
4-Methyl-m-phenylen-diisocyanat mit 1,4-Diaminobutan.

Die Isolierung der enzymhaltigen Polymere aus der Reaktionslösung erfolgt nach herkömmlichen Methoden der Abtrennung von Feststoffen aus fluiden Systemen, wie z.B. durch Abfiltrieren und Trocknen des ausfallenden enzymhaltigen Polymers. Vorzugsweise wird bei Verwendung von Lösungsmitteln, die zu klebrigen Polymeren führen, vor dem Abfiltrieren Methyl-t-butylether (MTBE) zugegeben, um rieselfähige Produkte zu erhalten. Ohne Zugabe von physikalischen Treibmitteln zu Beginn der Reaktion fallen die enzymhaltigen Polymere als amorphe Festkörper an. Zur Anpassung des Festkörpers an bestimmte Ausführungsformen von Reaktoren, kann der amorphe Festkörper nachbearbeitet, wie beispielsweise zerrieben und gepreßt werden.

Die Erfindung betrifft ferner enzymhaltige Polymere, erhältlich nach dem vorstehend beschriebenen Verfahren.

Die erfindungsgemäßen enzymhaltigen Polymere werden in chemischen Reaktionen als Katalysator verwendet. Als chemische Reaktionen werden erfindungsgemäß solche Reaktionen verstanden, die die Enzyme im nicht immobilisierten, freien Zustand in Lösung katalysieren können. Beispielhaft seien folgende Reaktionen erwähnt:
Acylierung oder enantioselektive Acylierung von Alkoholen, Acylierung oder enantioselektive Acylierung von Aminen und Aminoestern, Verseifung oder enantioselektive Verseifung von Estern,
Acylierung oder enantioselektive Acylierung von Cyanhydrinen, Verseifung oder enantioselektive Verseifung von Cyanhydrinestern, Asymmetrisierung von Meso-diolen, Asymmetrisierung von Mesodiestern durch Verseifung, Epoxidierung, Oxidation, Aldolreaktionen, Amidverseifungen, Spaltung und Abbau von Proteinen, Umesterungen oder Hydrolyse von Epoxiden.

Bevorzugt ist die Verwendung von erfindungsgemäßen enzymhaltigem Polymer, wie z.B. lipasehaltigem Polymer zur Katalyse der Acylierung oder enantioselektive Acylierung von Alkoholen. Besonders bevorzugt wird bei dieser Reaktion ein Polymer verwendet, welches Lipase von *Burgholderi Plantarii* enthält und durch Verwendung der vorstehend erwähnten, bevorzugten Kombination von mindestens bifunktionellen Monomeren und mindestens bifunktionellen Aminen erhalten wurde.

Das Verfahren zur enzymkatalytischen Umwandlung von Substraten ist dadurch gekennzeichnet, daß man die Substrate in Gegenwart des enzymhaltigen Polymers umsetzt. Je nachdem ob es der Reaktionstyp erfordert, werden dabei vorzugsweise weitere Reagenzien zugegeben. So erfordert z.B. eine Acylierung (wie Beispiel 2) die Zugabe eines Acylierungsmittels, während z.B. die Verseifung (wie Beispiel 4) keine Zugabe weiterer Reagenzien benötigt. Unter Substrat wird erfindungsgemäß eine chemische Verbindung verstanden, die von den nicht immobilisierten freien Enzymen in Lösung umgesetzt, d.h. chemisch verändert werden kann. Bei enantioselektiven Umwandlungen sind Stereoisomerengemische, von denen nur ein Stereoisomeres umgesetzt wird, ebenfalls Substrate. Beispielhaft seien Alkohole, Amine, Aminoester, Amide, Ester, Thioester, Thiole, Cyanhydrine, Cyanhydrinester, Meso-diole, Alkene, Proteine, Epoxide und Halohydrine erwähnt. Das Verfahren wird vorzugsweise in Lösung, bei flüssigen Substraten mit oder ohne Lösungsmittel durchgeführt. Als Lösungsmittel können flüssige Lösungsmittel wie Wasser oder organischen Lösungsmittel sowie auch wäßrig/organische Zweiphasengemische verwendet werden. Vorzugsweise werden als organische Lösungsmittel Dioxan, THF, Diethylether, Methyl-t-butylether (MTBE), Toluol oder Heptan verwendet. Als wäßrig/organisches Zweiphasengemisch wird vorzugsweise ein Wasser/MTBE-Gemisch in beliebigem Verhältnis eingesetzt. Bei Verfahrensdurchführung in Lösung ist die Substratkonzentration nicht kritisch, liegt aber vorzugsweise zwischen 0.5 Gew.-% und 50 Gew.-% bezogen auf die Lösung, besonders bevorzugt sind 20 bis 30 Gew.-%. Die Temperatur bei der Durchführung des Verfahrens ist ebenfalls nicht kritisch, ist aber nach oben durch die Temperaturstabilität des Enzyms im Polymer beschränkt. Vorzugsweise wird das Verfahren bei 0°C bis 60°C durchgeführt, besonders bevorzugt sind 15°C bis 40°C.

Das Verfahren kann kontinuierlich oder diskontinuierlich durchgeführt werden. Für die kontinuierliche Verfahrensdurchführung leitet man beispielsweise in an sich bekannter Weise in einem Reaktor durch eine Schüttschicht aus enzymhaltigen Polymer eine flüssige mobile Phase. Die mobile Phase kann entweder eine Lösung von Substrat (und Reagenzien) bzw. die flüssigen Substrate (und Reagenzien) ohne Lösungsmittel sein. Die Durchflußrate ist nicht kritisch und richtet sich nach verfahrenstechnischen Gesichtspunkten wie Höhe, Durchmesser und Korngröße der Schüttschicht sowie nach der Ausgestaltung des Reaktors. Die Korngröße des enzymhaltigen Polymers ist ebenfalls nicht kritisch bezüglich der Enzymaktivität. Als Reaktoren für das kontinuierliche Verfahren verwendet man vorzugsweise die für kontinuierliche, heterogenkatalytische Prozesse (Fluid/Feststoff-Reaktionen) üblichen Reaktoren (J. Hagen, Chemische Reaktionstechnik, VCH, Weinheim 1992, S. 165-169). Beispielhaft seien Wirbelschichtreaktoren und Festbettreaktoren, wie Rohrreaktor, Säulenreaktor, Vollraumreaktor, Hordenreaktor, Rohrbündelreaktor und Flachbett-Kontaktofen genannt.

In der diskontinuierlichen Verfahrensdurchführung werden die enzymhaltigen Polymere in an sich bekannter Weise in einem Reaktor in einer Lösung von Substrat (und Reagenzien) bzw. in flüssigen Substraten (und Reagenzien) mit oder ohne Lösungsmittel suspendiert und die Suspension durchmischt. Als Reaktoren für das diskontinuierliche Verfahren verwendet man vorzugsweise die für diskontinuierliche, heterogenkatalytische Prozesse (Fluid/Feststoff-Reaktionen) üblichen Reaktoren mit Schüttel-, Misch- oder Rührvorrichtung. Beispielhaft sei der Rührkessel und sich davon ableitende Ausgestaltungen sowie Reaktionsgefäße mit Schüttelvorrichtung erwähnt.

Nach Beendigung der Reaktion (Erreichung des thermodynamischen Gleichgewichts) wird das enzymhaltige Polymer, vorzugsweise durch Dekantieren oder Abfiltrieren und Waschen mit einem Lösungsmittel isoliert und in weiteren Reaktionen verwendet.

In einer bevorzugten Ausführungsform des Verfahrens werden Substrate, die acylierbare funktionelle Gruppen wie z.B. Hydroxyl-, Amino- oder Thiol-Gruppen enthalten, wie Alkohole, Amine oder Thiole mit Acylierungsmitteln in Gegenwart des enzymhaltigen Polymers als Katalysator acyliert.

Besonders bevorzugt ist ein Verfahren zur Acylierung oder enantioselektiven Acylierung von Alkoholen mit Acylierungsmitteln in Gegenwart eines Polymers als Katalysator, welches erfindungsgemäß Lipase von *Burgholderi Plantarii* enthält (siehe Beispiel 2).

Hinsichtlich der Alkohole gibt es praktisch keine Beschränkung.
So können ein- und mehrwertige Alkohole wie z.B.
1-Phenylethanol,
2-Chlor-1-phenylethanol,
2-Chlor-1-(m-chlorphenyl)-ethanol,
Pent-3-in-2-ol,
1-Butin-3-ol,
2-Hydroxy-4-phenylbuttersäureester,
a-Methyl-(1,3)-benzodioxol-5-ethanol,
2-propanol-1-(1,3-benzodioxol-4-yl),
trans-2-Methoxy-cyclohexanol oder
2-Methoxy-2-phenyl-ethanol verwendet werden.

Als Acylierungsmittel werden erfindungsgemäß organische Verbindungen verstanden, die in Gegenwart von acylierenden Enzymen, wie z.B. der Lipase von *Burgholderi Plantarii,* in Lösung als Acylüberträger fungieren können. Beispielhaft seien erwähnt:
Aliphatische, araliphatische oder aromatische Carbonsäuren die gegebenenfalls mit Halogen, wie Cl, Br, I, F substituiert sind (Acylierung), wie
C₁-C₆-Alkancarbonsäuren, z.B. Ameisensäure, Essigsäure, Propionsäure, Buttersäure oder
wie araliphatische oder aromatische Carbonsäuren z.B. Benzoesäure, 3-Phenylpropionsäure oder
die entsprechenden Carbonsäureester (Umesterung) wie beispielsweise
3-Phenylpropionsäureester oder Essigsäurealkylester, wie z.B. Essigsäureethylester.

Bevorzugte Carbonsäureester als Acylierungsmittel sind Vinylester der Formel III 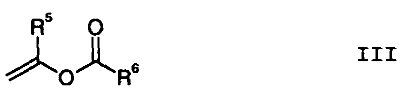 in der
- R⁵: Wasserstoff oder eine C₁-C₄-Alkyl-, vorzugsweise Methylgruppe und
- R⁶: Wasserstoff, C₁-C₁₈-Alkyl, welches gegebenenfalls mit Halogen substituiert ist, Phenyl oder (C₁-C₃-)Alkoxy-(C₁-C₄)-Alkyl bedeutet, wie Vinylformiat, Vinylacetat, Vinylpropionat oder Vinylbutyrat.

Acylierungsmittel sind weiterhin aliphatische, cycloaliphatische, araliphatische oder aromatische Carbonsäureanhydride und gemischte Carbonsäureanhydride (Acylierung) wie Essigsäureanhydrid, Bernsteinsäureanhydrid (BSA), Buttersäureanhydrid, 2-Ethylhexansäureanhydrid oder Methyl-Bernsteinsäureanhydrid.

Das Verfahren wird vorzugsweise entweder in Lösung oder bei flüssigen Substraten auch ohne Lösungsmittel durchgeführt. Als Lösungsmittel werden für dieses Verfahren vorzugsweise organische Lösungsmittel verwendet. Vorzugsweise werden als organische Lösungsmittel Ether, aromatische oder aliphatische Kohlenwasserstoffe wie Dioxan, THF, Diethylether, Methyl-t-butylether (MTBE). Toluol oder Heptan verwendet. Bei Verfahrensdurchführung in Lösung ist die Substratkonzentration nicht kritisch, liegt aber vorzugsweise zwischen 0.5 Gew.-% und 50 Gew.-% bezogen auf die Lösung, besonders bevorzugt sind 20 bis 30 Gew.-%. Im Falle der Verwendung von Bernsteinsäureanhydrid (BSA) oder anderer schlecht löslicher Anhydride als Acylierungsmittel kann Propylencarbonat besonders vorteilhaft zugemischt werden, um das BSA in Lösung zu bringen. Dies ist vor allem im Hinblick auf ein kontinuierliches Verfahren von Bedeutung.

Das Verfahren kann wie vorstehend erwähnt kontinuierlich oder diskontinuierlich durchgeführt werden. In dieser besonders bevorzugten Ausführungsform des Verfahrens wird im diskontinuierlichen Fall (siehe Beispiel 2.a) ein Alkohol und ein Acylierungsmittel wie vorherstehend erwähnt in einem aprotischen Lösungsmittel bzw. auch ohne Lösungsmittel vorgelegt und ein erfindungsmäßigen Polymer, welches Lipase von *Burgholderi Plantarii* enthält zugegeben. Die Suspension wird während der Reaktion durchmischt. Die Menge des zugegebenen Katalysators ist nicht kritisch, vorzugsweise beträgt die Menge eines freier Lipase entsprechenden Aliquots des Polymeren in einer typischen Ausführungsform ca. 0,01 bis 5 Gew.-%. des eingesetzten Alkohols als Substrat. Die Dauer der Umsetzung richtet sich nach der Einstellung des thermodynamischen Gleichgewichts und ist nicht kritisch. Im allgemeinen beträgt die Dauer der Umsetzung 6h bis 24h. Nach Beendigung der Reaktion wird das enzymhaltige Polymer, vorzugsweise durch Dekantieren oder Abfiltrieren und Waschen mit einem aprotischen Lösungsmittel isoliert und in weiteren Reaktionen verwendet.
Im kontinuierlichen Fall wird wie vorstehend beschrieben verfahren. Ein Beispiel hierzu ist in Beispiel 2.b beschrieben.

Das Verfahren zur enantioselektiven enzymkatalytischen Umwandlung von Substraten mit den erfindungsgemäßen Polymeren kann zur Abtrennung von Stereoisomeren und speziell zur Abtrennung von Enantiomeren aus dem Stereoisomerengemisch des Substrats verwendet werden. Das Verfahren zur enantioselektiven Acylierung von Alkoholen wird vorzugsweise zur Trennung von racemischen Alkoholen verwendet. Durch die enantioselektive Substratspezifität der immobilisierten Lipase, wird nur ein Enantiomeres des racemischen Alkohols acyliert, das andere Enantiomere bleibt übrig. Die entstehenden Produkte lassen sich durch chemische, physikalische und mechanische Trennmethoden in an sich bekannter Weise leicht trennen. Beispielhaft seien Kristallisation, Fällung, Extraktion in zweiphasigen Lösungsmittelsystemen und thermische Trennungsverfahren wie Destillation genannt.

Die lipasehaltigen Polymere können durch Zusatz von Wasser unter Verwendung eines vorzugsweise zweiphasigen Systems wie z.B. Wasser/MTBE auch die Verseifung von Estern katalysieren.

Die erfindungsmäßigen enzymhaltigen Polymere stellen wertvolle Katalysatoren für Umsetzungen und enantioselektive Umsetzungen dar. Gegenüber den freien Enzymen zeichnen Sie sich durch eine hohe Standzeit und leichte Isolierbarkeit aus den Reaktionsgemischen nach Beendigung der Reaktion aus. Gegenüber dem Stand der Technik haben die erfindungsmäßigen enzymhaltigen Polymere folgende Vorteile:
- Die Polymere können mit sehr viel höherer Dichte an aktiver Enzymspezies beladen werden. Bei gleichbleibendem Umsatz und gleichbleibender Standzeit werden damit bei enzymkatalytischen Reaktionen höhere Raum-Zeit-Ausbeuten (RZA) erreicht.
- Die Herstellung der enzymhaltigen Polymere aus organischer Lösung ist durch die Verwendungsmöglichkeit von Monomeren der Polyurethanchemie einfacher und damit wirtschaftlicher. Auf die Herstellung von amphiphilen Präpolymeren kann verzichtet werden.
- Die enzymhaltigen Polymere können sowohl als Schaum als auch als amorpher Festkörper hergestellt werden, so daß sie sich die Polymereigenschaften und damit die Enzymaktivitäten flexibel optimieren lassen.
- Die erfindungsgemäßen enzymhaltigen Polymere haben gegenüber dem Stand der Technik eine höhere Standzeit.

Die nachstehenden Beispiele erläutern die Erfindung:

### Beispiel 1

Herstellung enzymhaltiger Polymere am Beispiel der Lipase (*Burgholderi Plantarii*)
a) Immobilisierung der Lipase durch Einpolymerisation in ein Polyurethan aus Ethylendiamin und MDI (4,4'-Methylen-diphenyldiisocyanat) (Tabelle 1, 1A)
   500 mg Lipase (*Burgholderi Plantarii*) wurden in 100 ml Toluol suspendiert und mit 40 mmol MDI versetzt. Nach 1 Minute Rühren bei 0°C wurden 40 mmol Ethylendiamin zugegeben. Es wurde 15 min bei RT nachgerührt. Nach Zugabe von 200 ml MTBE wurde abgesaugt und der Feststoff getrocknet. Ausbeute : 10.5 g schwachgelber Feststoff.
b) bis x) Analog zu dieser Vorschrift wurden folgende enzymhaltige Polymere unter Verwendung der in den Zeilen angegebenen Diaminen (1 bis 6) und den in den Spalten angegebenen bifunktionellen Monomeren (A bis D) in den angegebenen Ausbeuten hergestellt. Alle enzymhaltigen Polymere wurden als schwachgelbe Feststoffe erhalten.

**Tabelle 1**

| Ausbeute an enzymhaltigen Polymer | | | | | |
|---|---|---|---|---|---|
| | | **A** | **B** | **C** | **D** |
| | | 4,4'-Methylen-diphenyl-diisocyanat | p-Phenylendiisocyanat | 4-Methyl-m-phenylendiisocyanat | p-Phenylendiisothiocyanat |
| **1** | 1,2-Diaminoethan | a) 10.5 g | b) 8.0 g | c) 8.5 g | d) 8.9 g |
| **2** | 1,2-Diaminobutan | e) 12.5 g | f) 9.2 g | g) 10.2 g | h) 10.0 g |
| **3** | N,N'-Diethylethylen-diamin | i) 14.3 g | j) 10.1 g | k) 11.4 g | l) 11.2 g |
| **4** | N-Phenyt-ethylendiamin | m) 15.0 g | n) 11.1 g | o) 15.3 g | p) 12.3 g |
| **5** | 1,4-Diaminobutan | q) 11.9 g | r) 9.9 g | s) 10.4 g | t) 10.9 g |
| **6** | 1,6-Diaminohexan | u) 14.0 g | v) 10.1 g | w) 10.6 g | x) 11.2 g |

### Beispiel 2

Enantioselektive Acylierung von racemischen 1-Phenyl-ethanol als Substrat mit Vinylpropionat als Acylierungsmittel in Gegenwart der nach Beispiel 1 hergestellten lipasehaltigen Polymere und Bestimmung der Standzeit

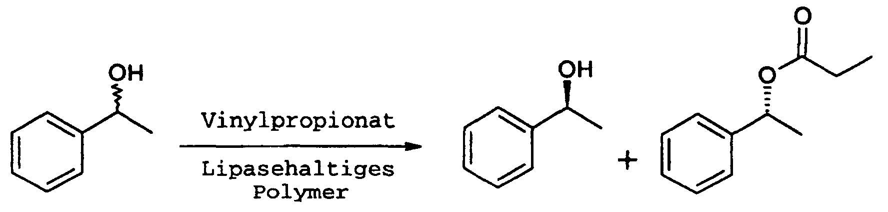

### Beispiel 2.a

### Diskontinuierliches Verfahren

Zunächst wurde eine Stammlösung aus 500 g (4.1 mol) 1-Phenylethanol und 246 g ( 2.46 mol) Vinylpropionat in 2 l MTBE bereitet. 5 ml dieser Lösung wurden zu jeweils einem 10 mg freier Lipase entsprechenden Aliquot des lipasehaltigen Polymeren gegeben. Es wurde 12 h bei RT geschüttelt, vom lipasehaltigem Polymer abfiltriert und der Umsatz und die Enantioselektivität des acylierten Antipoden per GC bestimmt und in Tabelle 2 aufgelistet. Die Filterrückstände wurden mit MTBE gewaschen und erneut eingesetzt, um die Standzeit zu bestimmen. Die Versuchsdurchführung wurde bis zu 10 mal wiederholt. Als lipasehaltige Polymere wurden die in Beispiel 1 hergestellten verwendet. Zum Vergleich wurde der analoge Versuch mit der freien Lipase durchgeführt.

**Tabelle 2.1:**

| | | |
|---|---|---|
| Umsatz (U in [%]) und Enantioselektivität (ee in [%]) des acylierten Antipoden bei der enantioselektiven Acylierung von racemischen 1-Phenyl-ethanol in Gegenwart der freien Lipase in einem diskontinuierlichen Verfahren. Das Verfahren wurde mit der aus dem vorhergehenden Versuch isolierten Lipase bis zu 10 mal wiederholt. | | |

| freie Lipase | | |
|---|---|---|
| Lauf | U | ee |
| 1 | 51.6 | 99 |
| 2 | 44.8 | 99 |
| 3 | 42.4 | 99 |
| 4 | 38.4 | 99 |
| 5 | 23.7 | 99 |
| 10 | 11.3 | 99 |

**Tabelle 2.2**

| | | | | | | | | | | | |
|---|---|---|---|---|---|---|---|---|---|---|---|
| Umsatz (U in [%]) und Enantioselektivität (ee in [%]) des acylierten Antipoden bei der enantioselektiven Acylierung von racemischen 1-Phenyl-ethanol in Gegenwart des jeweiligen lipasehaltigem Polymer in einem diskontinuierlichen Verfahren. Das Verfahren wurde mit den aus dem vorhergehenden Versuch isolierten Polymeren bis zu 10 mal wiederholt. | | | | | | | | | | | |

| a) A1 | | | b) B1 | | | c) C1 | | | d) D1 | | |
|---|---|---|---|---|---|---|---|---|---|---|---|
| Lauf | U | ee | Lauf | U | ee | Lauf | U | ee | Lauf | U | ee |
| 1 | 44.1 | 99 | 1 | 49.6 | 99 | 1 | 49.3 | 99 | 1 | 42.1 | 99 |
| 2 | 37.8 | 99 | 2 | 50.1 | 99 | 2 | 46.8 | 99 | 2 | 47.0 | 99 |
| 3 | 34.6 | 99 | 3 | 49.5 | 99 | 3 | 41.5 | 99 | 3 | 30.5 | 99 |
| 4 | - | - | 4 | 48.9 | 99 | 4 | - | - | 4 | - | - |
| 5 | - | - | 5 | 43.3 | 99 | 5 | - | - | 5 | - | - |
| 10 | - | - | 10 | - | - | 10 | - | - | 10 | - | - |

| e) A2 | | | f) B2 | | | g) C2 | | | h) D2 | | |
|---|---|---|---|---|---|---|---|---|---|---|---|
| Lauf | U | ee | Lauf | U | ee | Lauf | U | ee | Lauf | U | ee |
| 1 | 37.7 | 99 | 1 | 49.3 | 99 | 1 | 44.4 | 99 | 1 | 52.0 | 98 |
| 2 | 32.5 | 99 | 2 | 51.5 | 98 | 2 | 49.8 | 99 | 2 | 51.2 | 98 |
| 3 | - | - | 3 | 51.1 | 98 | 3 | 45.9 | 99 | 3 | 43.3 | 99 |
| 4 | - | - | 4 | 45.6 | 99 | 4 | 50.3 | 99 | 4 | 42.1 | 99 |
| 5 | - | - | 5 | 45.9 | 99 | 5 | 48.3 | 99 | 5 | - | - |
| 10 | - | - | 10 | 41.4 | 99 | 10 | 45.6 | 99 | 10 | - | - |

| i) A3 | | | j) B3 | | | k) C3 | | | l) D3 | | |
|---|---|---|---|---|---|---|---|---|---|---|---|
| Lauf | U | ee | Lauf | U | ee | Lauf | U | ee | Lauf | U | ee |
| 1 | 43.5 | 99 | 1 | 48.2 | 99 | 1 | 47.5 | 99 | 1 | 53.6 | 97 |
| 2 | 44.5 | 99 | 2 | 40.0 | 99 | 2 | 48.8 | 99 | 2 | 49.0 | 99 |
| 3 | - | - | 3 | 51.0 | 98 | 3 | 46.6 | 99 | 3 | 36.4 | 99 |
| 4 | - | - | 4 | 44.3 | 99 | 4 | 47.6 | 99 | 4 | - | - |
| 5 | - | - | 5 | - | - | 5 | 49.4 | 99 | 5 | - | - |
| 10 | - | - | 10 | - | - | 10 | 48.4 | 99 | 10 | - | - |

| m) A4 | | | n) B4 | | | o) C4 | | | p) D4 | | |
|---|---|---|---|---|---|---|---|---|---|---|---|
| Lauf | U | ee | Lauf | U | ee | Lauf | U | ee | Lauf | U | ee |
| 1 | 50.1 | 99 | 1 | 50.4 | 99 | 1 | 48.9 | 99 | 1 | 49.1 | 99 |
| 2 | 19.5 | 98 | 2 | 47.7 | 99 | 2 | 47.7 | 99 | 2 | 51.6 | 98 |
| 3 | - | - | 3 | 47.3 | 99 | 3 | - | - | 3 | 33.4 | 99 |
| 4 | - | - | 4 | 46.1 | 99 | 4 | - | - | 4 | - | - |
| 5 | - | - | 5 | 49.1 | 99 | 5 | - | - | 5 | - | - |
| 10 | - | - | 10 | - | - | 10 | - | - | 10 | - | - |

| q) A5 | | | r) B5 | | | s) C5 | | | t) D5 | | |
|---|---|---|---|---|---|---|---|---|---|---|---|
| Lauf | U | ee | Lauf | U | ee | Lauf | U | ee | Lauf | U | ee |
| 1 | 43.9 | 99 | 1 | 50.6 | 99 | 1 | 50.4 | 99 | 1 | 46.3 | 99 |
| 2 | 48.7 | 99 | 2 | 51.4 | 99 | 2 | 42.7 | 99 | 2 | 47.3 | 99 |
| 3 | 43.9 | 99 | 3 | 49.5 | 99 | 3 | 43.7 | 99 | 3 | 46.7 | 99 |
| 4 | 45.1 | 99 | 4 | 42.4 | 99 | 4 | 46.4 | 99 | 4 | 44.3 | 99 |
| 5 | 43.7 | 99 | 5 | - | - | 5 | 51.0 | 98 | 5 | 43.9 | 99 |
| 10 | 41.8 | 99 | 10 | - | - | 10 | 48.9 | 99 | 10 | - | 99 |

| u) A6 | | | v) B6 | | | w) C6 | | | x) D6 | | |
|---|---|---|---|---|---|---|---|---|---|---|---|
| Lauf | U | ee | Lauf | U | ee | Lauf | U | ee | Lauf | U | ee |
| 1 | 48.5 | 99 | 1 | 46.1 | 99 | 1 | 45.3 | 99 | 1 | 41.8 | 99 |
| 2 | 47.0 | 99 | 2 | 49.3 | 99 | 2 | 37.3 | 99 | 2 | 45.4 | 99 |
| 3 | 36.8 | 99 | 3 | 51.7 | 98 | 3 | 40.3 | 99 | 3 | 42.9 | 99 |
| 4 | - | - | 4 | 50.2 | 99 | 4 | 37.5 | 99 | 4 | 41.5 | 99 |
| 5 | - | - | 5 | 49.6 | 99 | 5 | - | - | 5 | - | - |
| 10 | - | - | 10 | 49.4 | 99 | 10 | - | - | 10 | - | - |

### Beispiel 2.b

### Kontinuierliches Verfahren

In eine Chromatographiesäule mit 1 cm Durchmesser wurde ein lipasehaltiges Polymer (B6, Beispiel 1.v)) eingefüllt, und es wurde mittels einer Pumpe ein auf 30-35 ml/h eingestellter Strom der Eduktlösung über das Immobilisat gepumpt. In regelmäßigen Abständen wurden Proben genommen, per GC der Umsatz und die Enantioselektivität bestimmt und in Tabelle 4 aufgelistet. Zum Vergleich wurde ein Lyophilisat der freien Lipase eingesetzt.

**Tabelle 2.3:**

| | | | | | | | | | | | | |
|---|---|---|---|---|---|---|---|---|---|---|---|---|
| Umsatz (U) und Enantioselektivität (ee) in [%], gemessen in regelmäßigen Zeitabständen bei einem kontinuierlichen Verfahren der enantioselektiven Acylierung von racemischen 1-Phenyl-ethanol. Die Werte des lipasehaltigen Polymers B6 (Beispiel 1.v)) werden mit den Werten der freien Lipase verglichen. | | | | | | | | | | | | |

| Zeit | 10 h | | 20 h | | 50 h | | 100 h | | 150 h | | 200 h | |
|---|---|---|---|---|---|---|---|---|---|---|---|---|
| | U | ee | U | ee | U | ee | U | ee | U | ee | U | ee |
| freie Lipase | 54.3 | 97 | 46.7 | 99 | 36.2 | 99 | 24.5 | 99 | 12.0 | 99 | 5.6 | 99 |
| Lipasehaltiges Polymer B6 | 51.5 | 98 | 50.2 | 99 | 48.7 | 99 | 49.5 | 99 | 47.9 | 99 | 48.9 | 99 |

### Beispiel 3

### Einfluß der Beladungsdichte auf die Aktivität der Immobilisate

Analog zu Beispiel 1.v) wurde ein lipasehaltiges Polymer B6 unter Verwendung von 500 mg (Beladung (Gew.-%) 5 %), 100 mg (Beladung (Gew.-%) 1 %) und 50 mg (Beladung (Gew.-%) 0,5 %) Lipase (*Burgholderi Plantarii*) hergestellt.

Analog zu Beispiel 2.1 wurde die Aktivität und Standzeit der lipasehaltigen Polymere bei der enantioselektiven Acylierung von Alkoholen bestimmt.

Dazu wurde zunächst eine Stammlösung aus 500 g (4.1 mol) 1-Phenyl-ethanol und 246 g (2.46 mol) Vinylpropionat in 2 l MTBE bereitet. 5 ml dieser Lösung wurden zu jeweils einem 10 mg freier Lipase entsprechenden Aliquot des lipasehaltigen Polymeren gegeben (Bei geringer beladenen Polymeren, entsprechend mehr Polymermenge). Es wurde 12 h bei RT geschüttelt, vom lipasehaltigem Polymer abfiltriert und der Umsatz und die Enantioselektivität des acylierten Antipoden per GC bestimmt und in Tabelle 5 aufgelistet. Die Filterrückstände wurden mit MTBE gewaschen und erneut eingesetzt, um die Standzeit zu bestimmen. Die Versuchsdurchführung wurde bis 5 mal wiederholt. Zum Vergleich wurde der analoge Versuch mit der freien Lipase durchgeführt.

**Tabelle 3:**

| | | | | | | | | |
|---|---|---|---|---|---|---|---|---|
| Umsatz (U in [%]) und Enantioselektivität (ee in [%]) des acylierten Antipoden bei der enantioselektiven Acylierung von racemischen 1-Phenyl-ethanol in Gegenwart des lipasehaltigem Polymer B6 mit unterschiedlicher Beladung (Gew.-%) an Lipase in einem diskontinuierlichen Verfahren. Das Verfahren wurde mit den aus dem vorhergehenden Versuch isolierten Polymeren 5 mal wiederholt. | | | | | | | | |

| | freie Lipase | | B6/50 mg Lip. | | B6/100 mg Lip. | | B6/500 mg Lip. | |
|---|---|---|---|---|---|---|---|---|
| Beladung | - | | 0.5 % | | 1 % | | 5 % | |
| Lauf | U | ee | U | ee | U | ee | U | ee |
| 1 | 51,6 | 99 | 45.3 | 99 | 47.8 | 99 | 46.1 | 99 |
| 2 | 44,8 | 99 | 42.3 | 99 | 50.3 | 99 | 49.3 | 99 |
| 3 | 42.4 | 99 | 39.5 | 98 | 52.5 | 97 | 51.7 | 98 |
| 4 | 38.4 | 99 | 40.2 | 99 | 51.3 | 98 | 50.3 | 99 |
| 5 | 23.7 | 99 | 39.5 | 99 | 49.6 | 99 | 48.9 | 99 |

### Beispiel 4

### Verseifung von Estern mit lipasehaltigem Polymer in zweiphasigen (organischen/wäßrigen) Lösungsmittelsystemen

10 g Propionsäure-1-Phenylethylester wurden in 100 ml MTBE/Wasser (1:1, V/V) vorgelegt. Anschließend wurden 10.5 g lipasehaltiges Polymer (A1) gemäß Beispiel 1a) zugegeben und der Umsatz nach 3 h per GC bestimmt. Nach Beendigung der Reaktion (3 h) wurde das lipasehaltige Polymer durch Abfiltrieren, Absaugen und Waschen mit MTBE aus der Reaktionslösung isoliert und in einer zweiten analogen Versuchsanordnung verwendet.

Zum Vergleich wurde eine analoge Versuchsreihe mit der freien Lipase durchgeführt. Dazu wurden 10 g Propionsäure-1-Phenylethylester in 100 ml MTBE/ Wasser ( 1:1, V/V) vorgelegt. Anschließend wurden 500 mg freie Lipase (Burgholderi Plantarii) zugegeben und der Umsatz nach 3h per GC bestimmt. Nach Beendigung der Reaktion (3h) wurde die Lipase durch Abfiltrieren und Absaugen aus der Reaktionslösung isoliert und in einer zweiten analogen Versuchsanordnung verwendet.

**Tabelle 4:**

| | | | |
|---|---|---|---|
| Umsatz (in [%]) bei der Verseifung von Propionsäure-1-phenylethylester in Gegenwart des lipasehaltigem Polymer A1 (Beispiel 1.a)) bzw. der freien Lipase in einem diskontinuierlichen Verfahren. Das Verfahren wurde mit dem isolierten Polymer bzw. mit der isolierten freien Lipase wiederholt. | | | |

| Lipasehaltiges Polymer (A1) | | freie Lipase | |
|---|---|---|---|
| Lauf | Umsatz (3h; [%]) | Lauf | Umsatz (3h; [%]) |
| 1 | 10.5 | 1 | 12.4 |
| 2 | 10.0 | 2 | 2.4 |

### Vergleichsbeispiel 1

### Herstellung eines enzymhaltigen Polymers durch Umsetzung einer wäßrigen Enzymsuspension mit Polyisocyanat-Präpolymeren am Beispiel der Lipase (Burgholderi Plantarii)

17.2 g B278-Präpolymer (Warenzeichen der Firma BASF AG; Präpolymer bestehend aus mit MDI gecappten PO- (Polypropylenoxid; 25 %) und EO- (Ethylenoxid; 75 %) Spacereinheiten (Die OH-Zahl beträgt ca. 6500)) wurden mit einem Ultrarührer gerührt. Anschließend wurde 1 ml einer 10 % Lösung (Gew.-%) von Lipase (*Burgholderi Plantarii*) in Wasser zugegeben. Unter Gasentwicklung begann die Schaumbildung, die nach etwa 30 min beendet war. Der Schaum wurde mit 100 ml MTBE gewaschen und getrocknet.

Das erhaltene Produkt wies im Experiment nach Beispiel 2 (enantioselektive, enzymatische Acylierung) keine Aktivität auf.

## Patentansprüche

1. Verfahren zur Herstellung eines enzymhaltigen Polymers durch Umsetzung des Enzyms mit einer quervernetzenden, endständig reaktive funktionelle Gruppen tragenden organischen Verbindung **dadurch gekennzeichnet, daß** man das Enzym in einem organischen Lösungsmittel im ersten Schritt mit einem mindestens bifunktionellen Monomer
R¹(X)ₛ(Y)ₜ
umsetzt, wobei
R¹ eine 2- bis 5-fach gebundene Alkan-, Alken-, Cycloalkan-, Cycloalken-, Aryl-, Arylalkan-, Diarylenether-, Diarylenthioether- oder Diarylamingruppe darstellt, wobei die cyclischen aromatischen oder nichtaromatischen Gruppen ihrerseits durch ein bis vier C₁-C₄-Alkyl-, C₁-C₄-Halogenalkyl- und/oder Halogenreste substituiert sein können,
und
X die funktionelle Gruppe Isocyanat (-NCO) und
Y die funktionelle Gruppe Isothiocyanat (-NCS)
mit s ≥ 0 und t ≥ 0 und 5 ≥ s+t ≥ 2
oder
X=Y die funktionelle Gruppe -COR³
mit 5 ≥ s+t ≥ 2 darstellt,
worin R³ eine durch die Amino-, Hydroxy- oder Thio-Funktionalität des Enzyms verdrängbare Abgangsgruppe darstellt
und man in einem 2. Schritt ein mindestens bifunktionelles Amin oder ein Gemisch mindestens bifunktioneller Amine zugibt.

2. Verfahren nach Anspruch 1, **dadurch gekennzeichnet, daß** man als bifunktionelles Monomer und als bifunktionelles Amin folgende Kombinationen verwendet:
p-Phenylendiisocyanat mit 1,n-Diaminoalkan,
4-Methyl-m-phenylen-diisocyanat mit 1,n-Diaminoalkan,
4,4'-Methylen-bis-phenyldiisocyanat(MDI) mit 1,n-Diaminoalkan,
p-Phenylendiisocyanat mit N-Phenylethylendiamin,
p-Phenylendiisocyanat mit 1,6-Diaminohexan,
4-Methyl-m-phenylen-diisocyanat mit N,N'-Diethyl-ethylendiamin oder
4-Methyl-m-phenylen-diisocyanat mit 1,4-Diaminobutan,
wobei n eine ganze Zahl von 2 bis 12 darstellt.

3. Verfahren nach Anspruch 1 oder 2, **dadurch gekennzeichnet, daß** man Enzyme verwendet, die in organischen Lösungsmitteln eine enzymatische Aktivität aufweisen.

4. Verfahren nach einem der Ansprüche 1 bis 3, **dadurch gekennzeichnet, daß** man Lipasen, Amidasen, Esterasen, Haloperoxidasen, Proteasen und Hydrolasen als Enzyme verwendet.

5. Verfahren nach einem der Ansprüche 1 bis 4, **dadurch gekennzeichnet, daß** die Zeit zwischen Schritt 1 und 2 nicht länger als 1 s bis 10 min beträgt.

6. Verfahren nach einem der Ansprüche 1 bis 5, **dadurch gekennzeichnet, daß** man der Reaktionslösung vor der Umsetzung ein physikalisches Treibmittel beimischt.

7. Enzymhaltiges Polymer, erhältlich nach einem Verfahren gemäß einem der Ansprüche 1 bis 6.

8. Verfahren zur enzymkatalytischen Umwandlung oder enantioselektiven Umwandlung von Substraten, **dadurch gekennzeichnet, daß** man die Substrate in Gegenwart des enzymhaltigen Polymers nach Anspruch 7 umsetzt.

9. Verfahren nach Anspruch 8, **dadurch gekennzeichnet, daß** man als Substrate Alkohole verwendet und diese acyliert oder enantioselektiv acyliert.

10. Verfahren nach Anspruch 9, **dadurch gekennzeichnet, daß** man als Substrate Stereoisomerengemische oder Racemate von Alkoholen verwendet, diese enantioselektiv acyliert und anschließend die Gemische auftrennt.

11. Verwendung des enzymhaltigen Polymers nach Anspruch 7 als Katalysator in chemischen Reaktionen.

12. Verwendung des enzymhaltigen Polymer Anspruch 7 als Katalysator für die Acylierung oder enantioselektive Acylierung von Alkoholen.

## Claims

1. A process for preparing an enzyme-containing polymer by reacting the enzyme with a crosslinking organic compound with terminal reactive functional groups, which comprises reacting the enzyme in an organic solvent in the first step with an at least bifunctional monomer
R¹(X)ₛ(Y)ₜ
where
R¹ is an alkane, alkene, cycloalkane, cycloalkene, arene, arylalkane, diaryl ether, diaryl thioether or diarylamine group which is bonded 2 to 5 times, it being possible for the aromatic or nonaromatic cyclic groups in turn to be substituted by one to four C₁-C₄-alkyl, C₁-C₄-haloalkyl and/or halogen radicals,
and
X is the isocyanate (-NCO) functional group and
Y is the isothiocyanate (-NCS) functional group
with s ≥ 0 and t ≥ 0 and 5 ≥ s+t ≥ 2
or
X=Y is the -COR³ functional group
with 5 ≥ s+t ≥ 2,
in which R³ is a leaving group which can be displaced by the amino, hydroxyl or mercapto functionality of the enzyme,
and adding in a 2nd step an at least bifunctional amine or a mixture of at least bifunctional amines.

2. A process as claimed in claim 1, wherein the following combinations are used as bifunctional monomer and bifunctional amine:
p-phenylene diisocyanate with 1,n-diaminoalkane,
4-methyl-m-phenylene diisocyanate with 1,n-diaminoalkane,
4,4'-methylenebisphenyl diisocyanate(MDI) with 1,n-diaminoalkane,
p-phenylene diisocyanate with N-phenylethylenediamine,
p-phenylene diisocyanate with 1,6-diaminohexane,
4-methyl-m-phenylene diisocyanate with N,N'-diethyl-ethylenediamine or
4-methyl-m-phenylene diisocyanate with 1,4-diaminobutane,
where n is an integer from 2 to 12.

3. A process as claimed in claim 1 or 2, wherein enzymes which have an enzymatic activity in organic solvents are used.

4. A process as claimed in any of claims 1 to 3, wherein lipases, amidases, esterases, haloperoxidases, proteases and hydrolases are used as enzymes.

5. A process as claimed in any of claims 1 to 4, wherein the time between step 1 and 2 is not more than 1 s to 10 min.

6. A process as claimed in any of claims 1 to 5, wherein, before the reaction, a physical blowing agent is mixed with the reaction solution.

7. An enzyme-containing polymer obtainable by a process as claimed in any of claims 1 to 6.

8. A process for the enzyme-catalyzed transformation or enantioselective transformation of substrates, which comprises reacting the substrates in the presence of the enzyme-containing polymer as claimed in claim 7.

9. A process as claimed in claim 8, wherein alcohols are used as substrates and are acylated or enantioselectively acylated.

10. A process as claimed in claim 9, wherein mixtures of stereoisomers or racemates of alcohols are used as substrates and are enantioselectively acylated, and the mixtures are then fractionated.

11. The use of the enzyme-containing polymer as claimed in claim 7 as catalyst in chemical reactions.

12. The use of the enzyme-containing polymer as claimed in claim 7 as catalyst for the acylation or enantioselective acylation of alcohols.

## Revendications

1. Procédé pour la préparation d'un polymère contenant une enzyme par réaction de l'enzyme avec un composé organique portant des groupes fonctionnels réactifs terminaux, créant un réseau transversal, **caractérisé par le fait qu'**on fait réagir l'enzyme dans un solvant organique dans une première étape avec un monomère au moins bifonctionnel
R¹(X)ₛ(Y)ₜ
tandis que
R¹ représente un groupe alcane, alcène, cycloalcane, cycloalcène, aryle, arylalcane, diarylèneéther, diarylènethioéther ou diarylamine, tandis que les groupes aromatiques ou non-aromatiques cycliques peuvent quant à eux être substitués par un à quatre restes alkyle en C₁-C₄, halogénoalkyle en C₁-C₄ et/ou halogéno,
et
X désigne le groupe fonctionnel isocyanate (-NCO) et
Y désigne le groupe fonctionnel isothiocyanate (-NCS)
avec s ≥ 0 et t ≥ 0 et 5 ≥ s+t ≥ 2
ou
X=Y représente le groupe fonctionnel -COR³
avec 5 ≥ s+t ≥ 2,
tandis que R³ représente un groupe séparable, pouvant être déplacé par la fonctionnalité amino, hydroxy ou thio de l'enzyme
et qu'on ajoute dans une 2ème étape une amine au moins bifonctionnelle ou un mélange d'amines au moins bifonctionnelles.

2. Procédé selon la revendication 1, **caractérisé par le fait qu'**on utilise comme monomère bifonctionnel et comme amine bifonctionnelle les combinaisons suivantes:
p-phénylènediisocyanate avec 1,n-diaminoalcane,
4-méthyl-m-phénylène-diisocyanate avec 1,n-diaminoalcane,
4,4'-méthylène-bis-phényldiisocyanate (MDI) avec 1,n-diaminoalcane,
p-phénylènediisocyanate avec N-phényléthylènediamine,
p-phénylènediisocyanate avec 1,6-diaminohexane,
4-méthyl-m-phénylène-diisocyanate avec N,N'-diéthyléthylènediamine, ou
4-méthyl-m-phénylène-diisocyanate avec 1,4-diaminobutane,
tandis que n représente un nombre entier de 2 à 12.

3. Procédé selon la revendication 1 ou 2, **caractérisé par le fait qu'**on utilise des enzymes qui présentent une activité enzymatique dans des solvants organiques.

4. Procédé selon l'une des revendications 1 à 3, **caractérisé par le fait qu'**on utilise comme enzymes des lipases, des amidases, des estérases, des halogénoperoxydases, des protéases et des hydrolases.

5. Procédé selon l'une des revendications 1 à 4, **caractérisé par le fait que** la durée entre l'étape 1 et l'étape 2 n'est pas supérieure à 1 s à 10 min.

6. Procédé selon l'une des revendications 1 à 5, **caractérisé par le fait qu'**on incorpore un agent propulseur physique dans la solution de réaction avant la réaction.

7. Polymère contenant des enzymes, pouvant être obtenu conformément au procédé selon l'une des revendications 1 à 6.

8. Procédé pour -la transformation enzymatique ou la transformation énantiosélective de substrats, **caractérisé par le fait qu'**on fait réagir les substrats en présence du polymère contenant des enzymes selon la revendication 7.

9. Procédé selon la revendication 8, **caractérisé par le fait qu'**on utilise des alcools comme substrat et on acyle ou on acyle énantiosélectivement ceux-ci.

10. Procédé selon la revendication 9, **caractérisé par le fait qu'**on utilise comme substrat des mélanges de stéréoisomères ou des racémates d'alcools, on acyle ceux-ci énantiosélectivement et on sépare ensuite les mélanges.

11. Utilisation du polymère contenant des enzymes selon la revendication 7 comme catalyseur dans des réactions chimiques.

12. Utilisation du polymère contenant des enzymes selon la revendication 7 comme catalyseur pour l'acylation ou l'acylation énantiosélective d'alcools.
